**Europäisches Patentamt**

**European Patent Office**

**Office européen des brevets**

(19)

(11) Numéro de publication : **0 336 958 B1**

# FASCICULE DE BREVET EUROPEEN

(12)

(45) Date de publication du fascicule du brevet :
**16.12.92 Bulletin 92/51**

(51) Int. Cl.⁵ : **A61K 37/66, A61K 31/17**

(21) Numéro de dépôt : **88909786.1**

(22) Date de dépôt : **06.10.88**

(86) Numéro de dépôt international :
**PCT/FR88/00496**

(87) Numéro de publication internationale :
**WO 89/03224 20.04.89 Gazette 89/09**

(54) **PRODUIT COMPRENANT A TITRE DE PRINCIPES ACTIFS: A) UN PEPTIDE AYANT UNE ACTIVITE DU TYPE DE CELLE DE L'INTERFERON GAMMA HUMAIN, B) L'HYDROXYUREE.**

(30) Priorité : **08.10.87 FR 8713886**

(43) Date de publication de la demande :
**18.10.89 Bulletin 89/42**

(45) Mention de la délivrance du brevet :
**16.12.92 Bulletin 92/51**

(84) Etats contractants désignés :
**AT BE CH DE FR GB IT LI LU NL SE**

(56) Documents cités :
**US-A-39 682 499
CHEMICAL ABSTRACTS, vol. 100, nr. 3, 16 janvier 1984, Columbus, OH (US); S.YAMA-MOTO et al., p. 25, no. 17324q***
**CHEMICAL ABSTRACTS, vol. 99, no. 7, 15 août 1983, Columbus, OH (US); M.NAMBA et al., pp. 25-26, no. 47628v***

(73) Titulaire : **ROUSSEL-UCLAF
35, Boulevard des Invalides
F-75007 Paris (FR)**

(72) Inventeur : **DAMAIS, Chantal
24-26, rue du Cotentin
F-75015 Paris (FR)**
Inventeur : **LANDO, Danielle
17-19, rue de la Plaine
F-75020 Paris (FR)**

(74) Mandataire : **Bourgouin, André
Département des Brevets ROUSSEL UCLAF
111, route de Noisy B.P. no 9
F-93230 Romainville (FR)**

## Description

L'invention concerne un produit renfermant à titre de principes actifs :
- a/. un polypeptide ayant une activité du type de celle de l'interféron gamma humain,
- b/. l'hydroxyurée.

L'hydroxyurée est un anti-métabolite utilisé dans le traitement de la leucémie myéloïde chronique qui agit en inhibant la ribonucléotide diphospho réductase et en bloquant les cellules en phase $G_1/S$ (Cancer Res. 28 p 1559 (1968) ; J. Biol. Chem. 254 p 253 (1979).

D'autre part, l'interféron gamma humain possède des propriétés de différenciation, à savoir qu'il pousse les cellules promyélocytaires dans une voie de différenciation vers les monocytes et macrophages et non vers les polynucléaires.

Des travaux ont suggéré l'intérêt potentiel de cette substance dans le traitement de la leucémie myéloïde chronique (Leukemia, 1, No1(January) 1987 p 52-57).

De nombreux produits cytostatiques, autres que l'hydroxyurée, ont été associés à l'interféron gamma recombinant humain pour mesurer l'effet antiprolifératif de ces associations sur des lignées cellulaires d'origine non leucémique mais issues d'autres cancers.

Les résultats obtenus avec de telles associations sont très divers.

En effet, on observe une inhibition cellulaire soit supérieure, soit inférieure à celle observée avec chacun des produits étudiés séparément.

Par exemple, l'effet antiprolifératif combiné de l'interféron gamma et de la 5-fluoro uracile ou du méthotrexate a été étudié sur des lignées cellulaires provenant de différents cancers (Int. - J. Cancer (1984), Oct. 15, Vol. 34, p. 495-500).

On peut citer également l'association difluorométhylornithine - interféron gamma dont l'effet a été mesuré sur des lignées cellulaires provenant de mélanomes ou de cancers du sein (J. Biol. Response Mod. 1985 Aug. Vol. 4 p. 391-5).

Les études réalisées sur des lignées cellulaires d'origine leucémique, notamment sur les cellules leucémiques promyélocytaires de type HL60, concernent l'interféron-gamma combiné dans le milieu de culture avec d'autres substances pour lesquelles des propriétés de différenciation sont aussi décrites, à savoir qu'elles poussent les cellules promyélocytaires dans une voie de différenciation vers les granulocytes ou monocytes plus matures de cette lignée.

Ces différentes substances sont par exemple l'acide rétinoïque (Blood, Vol. 69, No2 Fev, 1987 p. 501-507) ou la 1,25-dihydroxy vitamine $D_3$ (Exp. Hematol. 14: 998-1005, (1986)).

De même, le "tumor necrosis factor" ou TNF a été associé également avec l'interféron-gamma (J. Exp. Med, Vol. 64, octobre (1986) p. 1206-1225).

Ces études montrent une coopération, voire même une synergie entre l'effet de différenciation de l'interféron-gamma et celui de ces différentes substances.

Cependant, aucune de ces associations décrites ne peut être comparée à l'association de la présente demande, puisque l'hydroxyurée n'est en aucun cas un agent de différenciation cellulaire, mais seulement un agent cytostatique ou même cytotoxique.

Par ailleurs, l'hydroxyurée étant un antimétabolite intervenant dans la synthèse d'ADN, le résultat prévisible de l'effet de l'association de l'invention sur différentes lignées de cellules leucémiques est une inefficacité de l'interféron gamma.

Or, contre toute attente, le résultat visible sur différents critères de différenciation de cellules tumorales de type promyélocytaire HL-60 n'est jamais cette absence d'effet attendue.

En effet, les résultats obtenus montrent tout au contraire que l'hydroxyurée favorise l'action de l'interféron gamma sur les différents critères de différenciation. Ces critères sont l'apparition des récepteurs Fc sur les cellules différenciées et la capacité de ces cellules à produire des ions supéroxydes (test NBT) et à synthétiser des estérases non spécifiques.

L'apparition des antigènes d'histocompatibilité de classe II est un critère également retenu.

De plus, un effet additif et même synergique, est même clairement démontré, notamment lorsque sont mesurées l'apparition du récepteur Fc et l'augmentation de l'expression des antigènes de classe II (HLA-DR).

Par ailleurs, l'effet très important de l'hydroxyurée dans l'arrêt de la croissance cellulaire, n'est absolument pas modifié par la présence de l'interféron gamma et aucune augmentation de toxicité n'est observée.

Toutes ces études réalisées sur des cellules leucémiques promyélocytaires (HL-60) sont tout à fait représentatives de l'état de non différenciation des cellules dans la leucémie myéloïde chronique.

Les propriétés de l'association hydroxyurée interféron-gamma humain démontrées par les résultats très favorables donnés plus loin dans la partie expérimentale sur ce modèle, rendent ladite association apte à être utilisée dans le traitement de la leucémie myéloïde chronique.

L'invention concerne donc le produit comprenant à titre de principes actifs :
a/. un polypeptide ayant une activité du type de celle de l'interféron gamma humain,
b/. l'hydroxyurée

en tant que produit de combinaison, pour une utilisation simultanée, séparée ou étalée dans le temps, dans une méthode de traitement de la leucémie myéloïde chronique et des syndromes apparentés.

Le polypeptide ayant une activité d'interféron gamma humain est notamment celui comportant 143 acides aminés correspondant à l'interféron gamma naturel ou obtenu par recombinaison génétique, ce dernier ayant une méthionine du côté N-terminal.

Cette structure est décrite dans Rinderknecht et al. Journal of Biological Chemistry Vol. 259, N¤11, 1984 p. 6790-6797.

L'invention englobe toutes les associations renfermant des polypeptides dont la séquence en acides aminés est dérivée de celle de l'interféron gamma humain naturel à 143 acides aminés par suppresion, remplacement ou addition d'un ou plusieurs acides aminés.

Les syndromes apparentés à la leucémie myéloïde chronique sont par exemple la splénomégalie myéloïde, la thrombocytémie essentielle et les syndromes myélodysplasiques.

L'invention concerne notamment le produit comprenant à titre de de principes actifs :

a/. un polypeptide ayant une activité du type de celle de l'interféron gamma humain,
b/. l'hydroxyurée

en tant que produit de combinaison, pour une utilisation simultanée, séparée ou étalée dans le temps, dans une méthode de traitement de la splénomégalie myéloïde.

L'invention concerne notamment le produit de combinaison caractérisé en ce qu'il renferme de l'interféron gamma humain obtenu par recombinaison génétique, ayant une séquence identique à celle de l'interféron gamma naturel. Cet interféron est nommé rHu-IFN-g ci-après dans le texte.

L'invention concerne particulièrement le produit de combinaison caractérisé en ce que l'interféron gamma humain obtenu par recombinaison génétique ayant une séquence identique à celle de l'interféron gamma naturel est renfermé dans une composition pharmaceutique destinée à une administration parentérale et en ce que l'hydroxyurée est renfermée dans une composition pharmaceutique destinée à l'administration par voie orale.

L'invention a plus particulièrement pour objet le produit de combinaison caractérisé en ce que l'interféron gamma humain obtenu par recombinaison génétique, ayant une séquence identique à celle de l'interféron gamma naturel est renfermé dans une composition pharmaceutique destinée à être administrée par voie intra-veineuse ou sous-cutanée de préférence à la dose de 10 à 50 x 10⁶ U par jour ou trois fois par semaine par voie intraveineuse ou sous cutanée et en ce que l'hydroxyurée est renfermée dans une composition pharmaceutique destinée à être administrée par voie orale, de préférence à la dose de 30 à 60 mg/kg par 24 heures.

L'hydroxyurée et l'interféron gamma humain recombinant peuvent être administrés soit simultanément, soit séparément par des voies différentes, soit de manière étalée dans le temps par exemple dans la même journée ou à un intervalle allant de quelques jours à quelques semaines, voire quelques mois. Dans ce dernier cas, parmi les nombreux schémas thérapeutiques pouvant être envisagés, on peut choisir de débuter le traitement uniquement avec de l'hydroxyurée à une dose de 30 à 60 mg/Kg/jour par voie orale, puis de diminuer cette posologie jusqu'à une dose de 15 à 30 mg/kg/jour, et d'associer ensuite une injection d'interféron gamma recombinant humain de 20 x 10⁶ unités/jour pendant plusieurs semaines ou plusieurs mois.

La dose d'hydroxyurée pourra être diminuée en fonction de la formule sanguine et notamment de la leucocytose.

L'invention concerne tout particulièrement le produit de combinaison caractérisé en ce que le rHu-IFN-g et l'hydroxyurée sont renfermés dans des compositions pharmaceutiques destinées à être administrées simultanément.

Dans ce cas, les doses utilisées pour les deux produits de l'association varient en fonction de l'état du malade.

Elles sont de 15 à 30 mg/kg/jour pour l'hydroxyurée par voie orale et de 20 x 10⁶ unités/jour de rHu-IFN-g par voie sous-cutanée ou intra-veineuse.

Par ailleurs, l'invention concerne aussi une composition pharmaceutique sous forme de kit renfermant :

- un soluté injectable renfermant une quantité active de rHu-IFN-g,
- un comprimé renfermant une quantité active d'hydroxyurée.

Les exemples donnés ci-après illustrent l'invention sans toutefois la limiter.

**Etude concernant la différenciation de cellules tumorales promyélocytaires HL-60 par l'interféron gamma humain obtenu par recombinaison génétique ayant une séquence identique à celle de l'interféron gamma naturel (rHu-IFN-g) associé à l'hydroxyurée.**

Mise en cultures des cellules :

Les cellules sont mises en culture dans du milieu RPMI 1640 (Seromed, BIOPRO) enrichi avec 10 % du sérum de veau foetal (Irvine Scientific, Cliniscience) du tampon HEPES et de la glutamine, ainsi que des antibiotiques (pénicilline/streptomycine et gentamycine)/ Les cellules après comptage (compte viable à l'aide de bleu trypan) sont mises en culture à $5.10^5$ cellules/ml en présence de différentes quantités d'rHu-IFN-g et d'hyroxyurée ajoutés en même temps dans le milieu de culture.

Au bout de 3 à 5 jours de stimulation, les cellules sont comptées et observées au microscope afin de vérifier leur état de différenciation d'un point de vue morphologique. En même temps, différentes expériences sont réalisées afin de connaître la différenciation des cellules au niveau fonctionnel.

Quatre critères ont été retenus :
- apparition des antigènes d'histocompatibilité de classe II (HLA-DR);
- apparition des récepteurs Fc ;
- capacité à produire des ions supéroxydes (test Nitro Blue tétrazolium : NBT) ;
- capacité à synthétiser des estérases non spécifiques.

1/. Antigènes HLA-DR :

- une technique d'essai radio immunologique a été utilisée avec un anticorps monoclonal (Becton Dickinson, réf. 7360) incubé au 1/80 avec $10^6$ cellules stimulées pendant 1 heure à 4¤C. La révélation de la fixation se fait avec un anticorps anti-Ig de souris marqué à l'iode 125.

On mesure l'expression membranaire des antigènes de classe II exprimés par le système majeur d'histocompatibilité.

Le résultat est exprimé en pourcentage d'augmentation de cellules positives.

2/. Récepteurs Fc :

Les récepteurs pour la partie Fc des immunoglobulines ont été mis en évidence par la méthode des rosettes utilisant des globules rouges de mouton recouverts d'immunoglobulines (rosettes E.A.) suivant la méthode dérivée de celle décrite en détails dans Parant M., Vinit M.-A., Damais C., Riveau G. et Chedid L., Exp. Hematol., 1985, 13, 221.

On mesure le pourcentage de cellules présentant les récepteurs Fc.

3/. Test NBT :

La technique utilisée est dérivée de celle décrite dans Pick E., Charon J., et Mizel D., J. Reticuloendoth. Soc., 1981, 30, 581.

Les cellules stimulées ou non sont incubées pendant 30 minutes avec du 4 béta-phorbol 12-myristate 13-acétate (Sigma) à 200 nM et du NBT à la dilution de 1 mg/ml. Le pourcentage de cellules positives est évalué après étalement des cellules sur lame réalisé à l'aide d'une cytocentrifugeuse. On mesure un pourcentage de cellules positives dont le cytoplasme est rempli de précipités bleus de NBT réduit sous forme de formazan.

4/. Mise en évidence des estérases non spécifiques :

La technique utilisée est dérivée de celle décrite dans Li C.Y., Lam K.W., et Yam L.T., J. Histochem. Cytochem., 1973, 21, 1.

On utilise un kit Sigma avec de l'alpha-naphtyl acétate comme substrat sur les cellules différenciées après étalement sur lame réalisé à l'aide d'une cytocentrifugeuse. On mesure un pourcentage de cellules positives dont le cytoplasme apparaît noir.

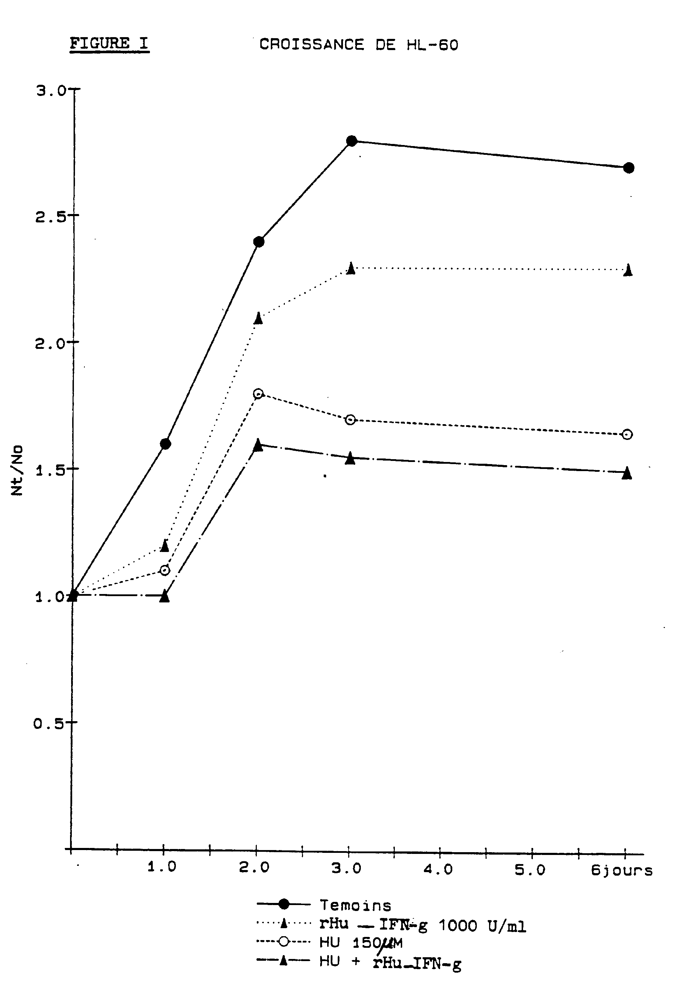

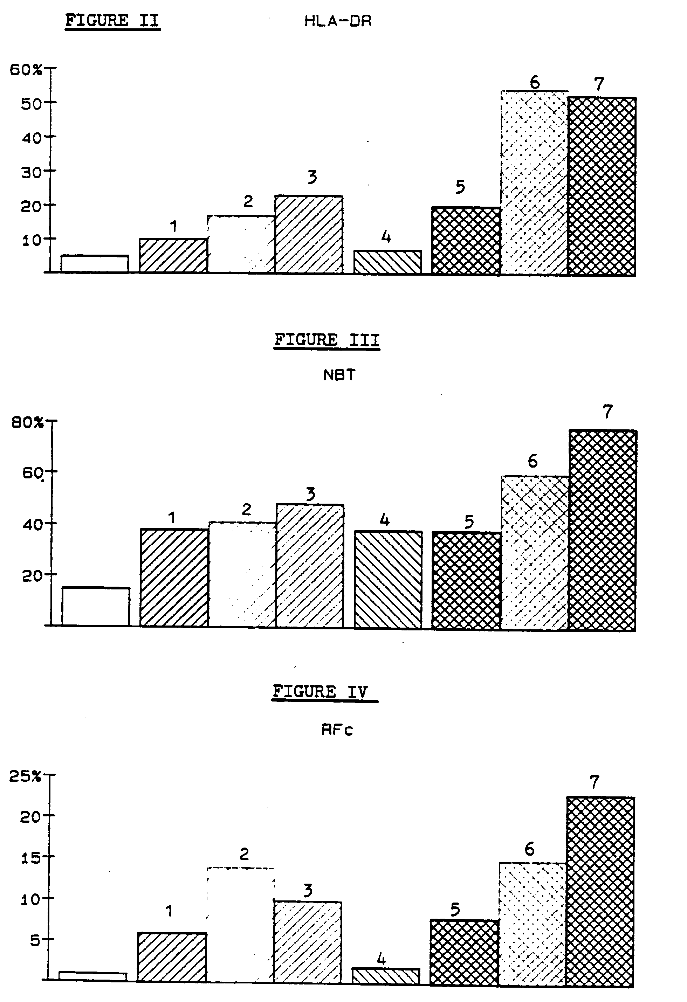

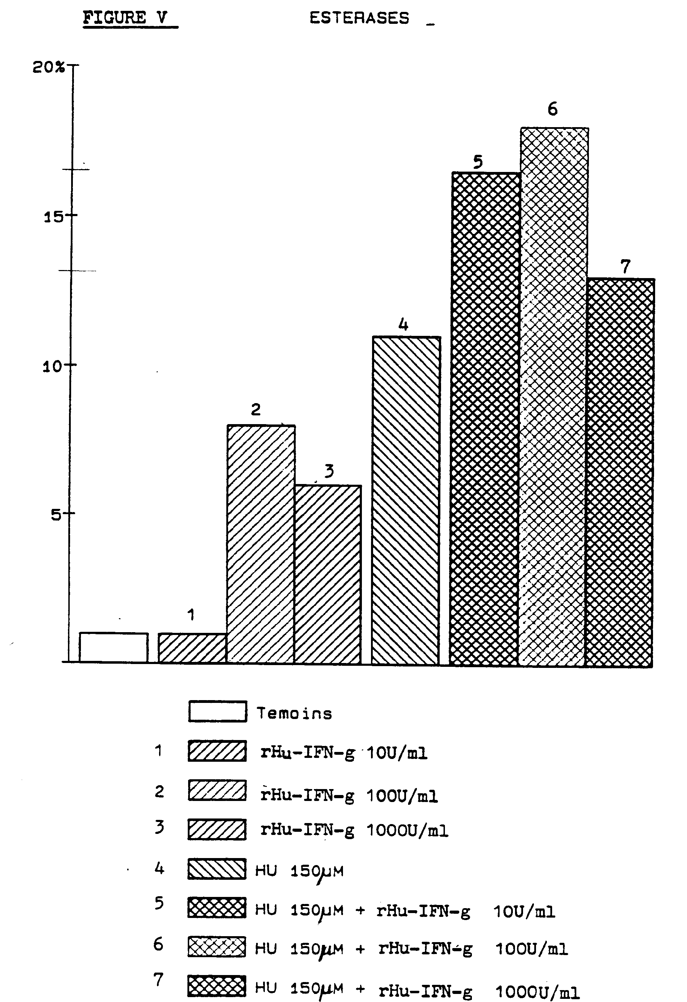

La figure I montre l'effet de 1000 U rHu-IFN-g/ml associé à 150 uM d'hydroxyurée sur la croissance des cellules HL-60.

La dose de 150 uM est choisie car cette dose est celle qui assure une inhibition suffisante de la croissance des cellules HL-60 sans être toxique.

La figure I met en évidence que l'IFN-g ne modifie absolument pas l'effet propre de l'hydroxyurée sur l'arrêt de la croissance cellulaire.

Les figures II, III, IV, et V montrent l'effet de l'association sur les différents critères de différenciation.

Cet effet est toujours au moins égal à celui obtenu avec le rHu-IFN-g ou l'hydroxyurée ajoutés seuls dans le milieu de culture.

Par ailleurs, un effet additif et même synergique a pu être mis en évidence.

## Revendications

1. Produit comprenant à titre de principes actifs :
   a) un polypeptide ayant une activité du type de celle de l'interféron gamma humain,
   b) l'hydroxyurée,
   en tant que produit de combinaison synergique, pour une utilisation simultanée, séparée ou étalée dans le temps, dans une méthode de traitement de la leucémie myéloïde chronique et des syndromes apparentés.

2. Produit comprenant à titre de principes actifs :
   a) un polypeptide ayant une activité du type de celle de l'interféron gamma humain,
   b) l'hydroxyurée
   en tant que produit de combinaison synergique, pour une utilisation simultanée, séparée ou étalée dans le temps, dans une méthode de traitement de la splénomégalie myéloïde.

3. Produit selon la revendication 1 ou 2, caractérisé en ce qu'il renferme de l'interféron gamma humain obtenu par recombinaison génétique ayant une séquence identique à celle de l'interféron gamma naturel (= rHu-IFN-g).

4. Produit selon les revendications 1 à 3, caractérisé en ce que l'interféron gamma humain obtenu par recombinaison génétique ayant une séquence identique à celle de l'interféron gamma naturel est renfermé dans une composition pharmaceutique destinée à une administration parentérale et en ce que l'hydroxyurée est renfermée dans une composition pharmaceutique destinée à l'administration par voie orale.

5. Produit selon les revendications 1 à 4, caractérisé en ce que l'interféron gamma humain obtenu par recombinaison génétique, ayant une séquence identique à celle de l'interféron gamma naturel est renfermé dans une composition pharmaceutique destinée à être administrée par voie intraveineuse ou sous-cutanée de préférence à la dose de 10 à 50 x 10⁶ U par jour ou trois fois par semaine par voie intraveineuse ou sous-cutanée.

6. Produit selon les revendications 1, 2 et 5, caractérisé en ce que l'hydorxyurée est administrée par voie orale, de préférence à la dose de 30 à 60 mg/kg par 24 h.

7. Produit selon les revendications 1 à 6, caractérisé en ce que l'interféron gamma humain obtenu par recombinaison génétique ayant une séquence identique à celle de l'interféron gamma naturel et l'hydroxyurée sont renfermés dans des compositions pharmaceutiques destinées à être administrées simultanément.

8. Composition pharmaceutique sous forme de kit renfermant :
   - un soluté injectable renfermant une quantité active d'interféron gamma humain obtenu par recombinaison génétique ayant une séquence identique à celle de l'interféron gamma naturel,
   - un comprimé renfermant une quantité active d'hydroxyurée.

**Patentansprüche**

1. Produkt, enthaltend als Wirkstoffe:
   a) ein Polypeptid mit einer Aktivität vom Typ derjenigen des menschlichen gamma-Interferons,
   b) Hydroxyharnstoff,
   als synergistisches Kombinationsprodukt zur gleichzeitigen getrennten oder zeitlich aufgeteilten Anwendung bei einer Methode zur Behandlung der chronische myeloischen Leukämie und verwandter Sydrome.

2. Produkt, enthaltend als Wirkstoffe:
   a) ein Polypeptid mit einer Aktivität vom Typ derjenigen des menschlichen gamma-Interferons,
   b) Hydroxyharnstoff
   als synergistisches Kombinationsprodukt für die gleichzeitige, getrennte oder zeitlich aufgeteilte Anwendung bei einer Methode zur Behandlung der myeloischen Splenomegalie.

3. Produkt gemäß Anspruch 1 oder 2, dadurch gekennzeichnet, daß es menschliches gamma-Interferon enthält, das durch genetische Rekombination erhalten wurde mit einer Sequenz, die identisch derjenigen des natürlichen gamma-Interferons (= rHu-IFN-g) ist.

4. Produkt gemäß den Ansprüchen 1 bis 3, dadurch gekennzeichnet, daß das menschliche gamma-Interferon, das durch genetische Rekombination erhalten wurde, mit einer Sequenz identisch derjenigen des natürlichen gamma-Interferons in einer pharmazeutischen Zusammensetzung enthalten ist, die zur parenteralen Verabreichung bestimmt ist, und daß der Hydroxyharnstoff in einer pharmazeutischen Zusammensetzung enthalten ist, die zur oralen Verabreichung bestimmt ist.

5. Produkt gemäß den Ansprüchen 1 bis 4, dadurch gekennzeichnet, daß das menschliche gamma-Interferon, das durch genetische Rekombination erhalten wurde, mit einer Sequenz, die identisch derjenigen des natürlichen Interferons ist, in einer pharmazeutischen Zusammensetzung enthalten ist, die zur intravenösen oder subkutanen Verabreichung bestimmt ist, vorzugsweise in einer Dosis von 10 bis 50 x 10⁶ Einheiten pro Tag oder dreimal pro Woche, durch intravenöse oder subkutane Verabreichung.

6. Produkt gemäß den Ansprüchen 1, 2 und 5, dadurch gekennzeichnet, daß der Hydroxyharnstoff auf oralem Wege vorzugsweise in einer Dosis von 30 bis 60 mg/kg pro 24 Stunden verabreicht wird.

7. Produkt gemäß den Ansprüchen 1 bis 6, dadurch gekennzeichnet, daß das menschliche gamma-Interferon, erhalten durch genetische Rekombination, mit einer Sequenz, die identisch derjenigen des natürlichen gamma-Interferons ist, und der Hydroxyharnstoff in pharmazeutischen Zusammensetzungen enthalten sind, die gleichzeitig verabreicht werden.

8. Pharmazeutische Zusammensetzung in Form eines Kits, enthaltend
   - einen injizierbaren gelösten Stoff, enthaltend eine aktive Menge menschliches gamma-Interferon, das durch genetische Rekombination erhalten wurde, mit einer Sequenz, die identisch mit derjenigen des natürlichen gamma-Interferons ist,
   - eine Tablette, die eine aktive Menge an Hydroxyharnstoff enthält.

**Claims**

1.  Product containing as active ingredients:
    a) a polypeptide having an activity of the type of that of human gamma interferon,
    b) hydroxyurea,
    as a synergic combination product, for use which is simultaneous, separate or spread out over time, in a treatment method for chronic myeloid leukaemia and similar syndromes.

2.  Product containing as active ingredients:
    a) a polypeptide having an activity of the type of that of human gamma interferon,
    2) hydroxyurea
    as a synergic combination product, for use which is simultaneous, separate or spread out over time, in a treatment method for myeloid spleno-megalia.

3.  Product according to claim 1 or 2, characterized in that it contains human gamma interferon obtained by genetic recombination having a sequence identical to that of natural gamma interferon (= rHu-IFN-g).

4.  Product according to claims 1 to 3, characterized in that the human gamma interferon obtained by genetic recombination having a sequence identical to that of natural gamma interferon is contained in a pharmaceutical composition intended for parenteral administration and in that the hydroxyurea is contained in a pharmaceutical composition intended for administration by oral route.

5.  Product according to claims 1 to 4, characterized in that the human gamma interferon obtained by genetic recombination, having a sequence identical to that of natural gamma interferon is contained in a pharmaceutical composition intended to be administered by intravenous or subcutaneous route preferably at a dose of 10 to 50 x $10^6$ U per day or three times per week by intravenous or subcutaneous route.

6.  Product according to claims 1, 2 and 5, characterized in that the hydroxyurea is administered by oral route, preferably at a dose of 30 to 60 mg/kg per 24 hours.

7.  Product according to claims 1 to 6, characterized in that the human gamma interferon obtained by genetic recombination having a sequence identical to that of natural gamma interferon and the hydroxyurea are contained in pharmaceutical compositions intended to be administered simultaneously.

8.  Pharmaceutical composition in the form of a kit containing:
    - an injectable solute containing an active quantity of human gamma interferon obtained by genetic recombination having a sequence identical to that of natural gamma interferon,
    - a tablet containing an active quantity of hydroxyurea.

FIGURE I          CROISSANCE DE HL-60

FIGURE II                    HLA-DR

FIGURE III

NBT

FIGURE IV

RFc

## FIGURE V          ESTERASES

Legend:

☐ Temoins

1 ▨ rHu-IFN-g 10U/ml

2 ▨ rHu-IFN-g 100U/ml

3 ▨ rHu-IFN-g 1000U/ml

4 ▨ HU 150μM

5 ▨ HU 150μM + rHu-IFN-g 10U/ml

6 ▨ HU 150μM + rHu-IFN-g 100U/ml

7 ▨ HU 150μM + rHu-IFN-g 1000U/ml